Europäisches Patentamt

European Patent Office

Office européen des brevets

(19)

(11) Publication number: 0 306 113
A1

## (12) EUROPEAN PATENT APPLICATION

(21) Application number: 88201901.1

(22) Date of filing: 02.09.88

(51) Int. Cl.4: **C07C 31/04 , C07C 29/15 , C07C 29/136 , C07C 69/06 , C07C 67/36**

(30) Priority: 04.09.87 EP 87201679

(43) Date of publication of application:
08.03.89 Bulletin 89/10

(84) Designated Contracting States:
BE DE FR GB IT NL

(71) Applicant: SHELL INTERNATIONALE
RESEARCH MAATSCHAPPIJ B.V.
Carel van Bylandtlaan 30
NL-2596 HR Den Haag(NL)

(72) Inventor: Sie, Swan Tiong
Badhuisweg 3
NL-1031 CM Amsterdam(NL)
Inventor: Drent, Eit
Badhuisweg 3
NL-1031 CM Amsterdam(NL)

(74) Representative: Aalbers, Onno et al
P.O. Box 302
NL-2501 CH The Hague(NL)

(54) Process for the manufacture of methanol.

(57) A process for the manufacture of methanol comprising reaction of a mixture comprising carbon monoxide and hydrogen in the presence of a catalyst system obtainable by combination of at least:
(a) a nickel salt,
(b) an alcohol and/or an alcoholate from an alkaline metal or alkaline earth metal, and
(c) a hydride of an alkali metal or an alkaline earth metal,
and allowing the combined components (a), (b) and (c) to react, in at least two separate interconnected reaction zones, between which the catalyst system is circulated, into which carbon monoxide/hydrogen mixtures of different composition are introduced and which are operated under different process conditions, such as temperature, pressure and feed gas mixture composition.

EP 0 306 113 A1

## PROCESS FOR THE MANUFACTURE OF METHANOL

The invention relates to a process for the manufacture of methanol from carbon monoxide and hydrogen and more particularly to the manufacture of methanol by means of catalyst systems obtainable by combination of at least

(a) a nickel salt,

(b) an alcohol and/or an alcoholate, derived from an alkali metal or alkaline earth metal, and

(c) a hydride of an alkali metal or alkaline earth metal, and allowing the combined components (a), (b) and (c) to react.

Embodiments of such a process are in principle known from the US patent specifications Nos. 4,613,623, 4,614,749, 4,619,946 and 4,623,634.

In these patents there is commonly disclosed a low temperature methanol preparation process, in which synthesis gas is directly converted to methanol using a catalyst system comprising sodium hydride, sodium alkanolate and nickel (II) acetate, optionally in combination with a metal carbonyl of a group VI metal and more particularly Mo, Cr or W metal, while as most preferred catalyst precursor is proposed a combination of nickel (II) acetate, sodium tert-amyl alcoholate and/or tert-amyl alcohol and sodium hydride.

Although these catalyst systems on laboratory scale might enable the preparation of methanol in improved yields and under more economical operational conditions (lower temperatures and pressures) as compared to the currently used industrial scale methanol manufacturing processes (operating at high pressures and temperatures, which involve high, economically unattractive equipment and operational costs) they were not applied up till now to industrial scale processes for methanol manufacture.

Due to the great demand for cheap methanol in very large amounts for application as fuel and as starting material for further chemical syntheses, there is an urgent need for an economically more attractive industrial bulk manufacturing process of methanol, starting from cheap starting materials and operating at attractive economical and environmental and safety conditions, i.e. using rather simple equipment and giving a significant reduction of the methanol cost price. An object of the present invention is therefore the development of such an industrial process for methanol manufacture.

As result of extensive research and development a process was now found, which meets the hereinbefore indicated requirements. The process comprises reaction of a mixture comprising carbon monoxide and hydrogen in the presence of a catalyst system obtainable by combination of at least:

(a) a nickel salt,

(b) an alcohol and/or an alcoholate from an alkaline metal or alkaline earth metal,

(c) a hydride of an alkali metal or an alkaline earth metal, and allowing the combined components (a), (b) and (c) to react, in at least two separate interconnected reaction zones, between which the catalyst system is circulated, into which mixtures comprising carbon monoxide and hydrogen of different composition are introduced and which are operated under different process conditions.

More particularly the process comprises two reaction zones, in one of which predominantly formate, preferably an alkyl formate e.g. methyl formate, is formed by reaction of a mixture comprising carbon monoxide and hydrogen which is relatively richer in carbon monoxide as compared to the stoichiometric consumption ratio $H_2:CO = 2$, i.e. a $H_2:CO$ molar ratio in the range of from 0.5 to 1.9 and more preferably from 1.0 to 1.8 (formate formation zone), while in the other reaction zone predominantly formate is hydrogenated to methanol with a mixture comprising hydrogen and carbon monoxide which is relatively richer in hydrogen, i.e. a $H_2:CO$ molar ratio in the range of from 2.5 to 4.5 and more preferably from 2.9 to 3.5 (formate hydrogenation zone).

Unconverted synthesis gas from the formate formation zone may be fed to the formate hydrogenation zone. However, the gas feed may be additionally changed depending on the desired conditions.

In case a single synthesis gas stream is to be converted to methanol, a synthesis gas stream of higher $H_2/CO$ ratio is obtained by partial conversion of the first mentioned stream to form mainly the formate ester. The unconverted hydrogen enriched residual gas stream from the first zone is used to hydrogenate this formate ester in the second zone.

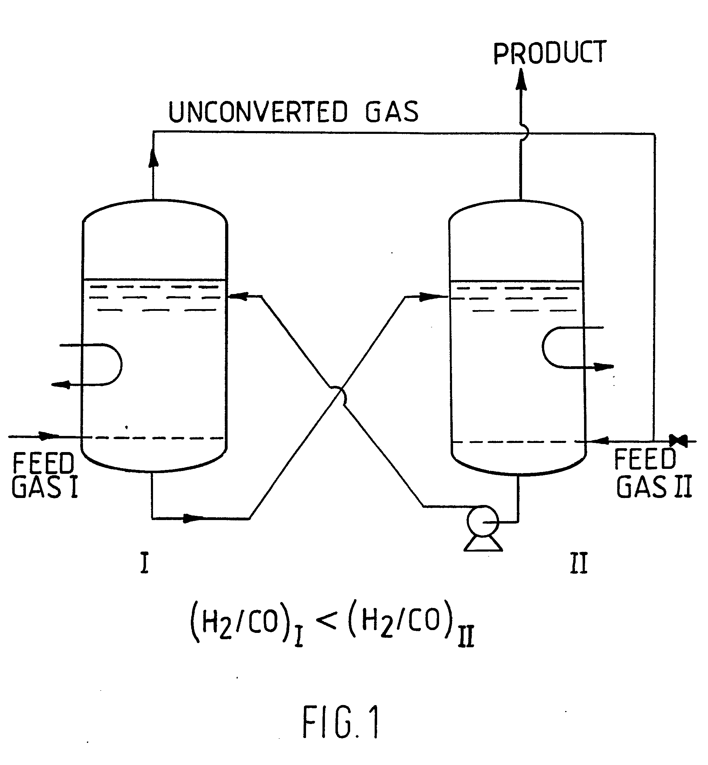

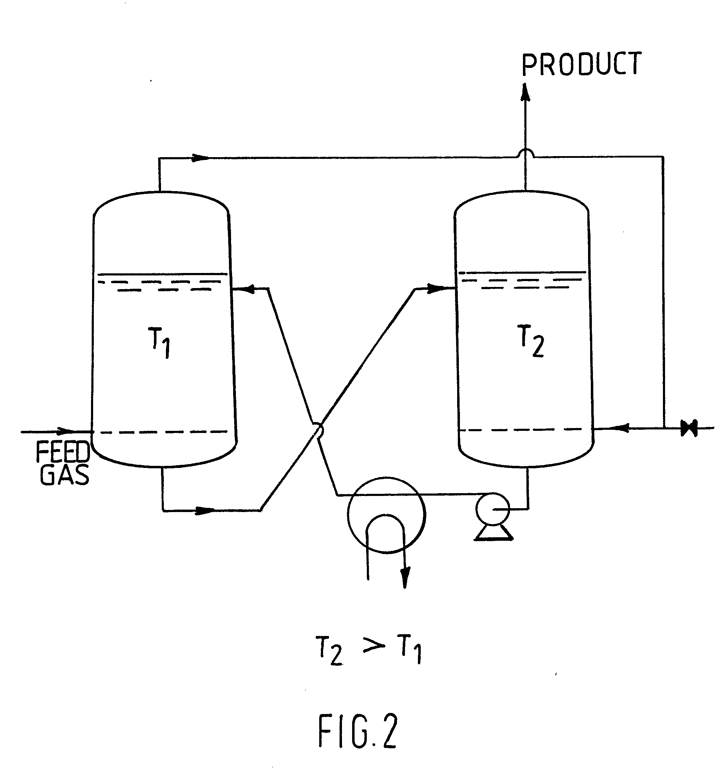

Typical embodiments of the process may be carried out in the equipment as depicted in figures 1 and 2, wherein both reactors for the separate reaction steps and the involved gas and liquid flows have been indicated.

According to a more preferred embodiment of the process, different temperatures may be maintained in both separated reaction zones in order to obtain optimization for both reaction steps. The formate formation (carbonylation) reaction step is preferably carried out at relatively low temperature, while the hydrogenation step is carried out at relatively higher temperature.

According to a most preferred embodiment, the both gas feeds to the two separate reaction zones will be derived from natural gas by its conversion into a synthesis gas, relatively rich in carbon monoxide, by partial oxidation, and by its conversion into a synthesis gas, relatively rich in hydrogen, by steam reforming.

At a substantial conversion of the low $H_2/CO$ ratio gas in the carbonylation reaction step these will be a net formate production with a $H_2/CO$ consumption ratio less than 2.

In the other reaction zone, where the hydrogenation step takes place, substantial conversion of high $H_2/CO$ ratio gas with a net consumption of formate takes place.

It will be appreciated that one of the features of the present invention is formed by the conception that formate production and consumption may be effectively balanced by control of the circulation of the liquid catalyst system (liquid and/or slurry).

It will be appreciated that the process of the present invention, reducing the concept of a two step process into practice contrary to the continuing efforts by skilled people aiming at optimization of a single stage process, based on opposite earlier theories about the mechanisms of formation of methanol and methylformate and showing attractive yields and being operable under mild conditions (low temperature and pressure), could certainly not be predicted or expected by people skilled in the art.

E.g. in this connection reference is made to page 2, lines 17-26 of the published patent application WO 84/00360 and to the before mentioned prior art references.

Although it clearly seemed to be advantageous to persons skilled in the art, to aim at a single step (one pot) process, as being the most simple and straightforward way, the following negative features of such single step process may be appreciated:

1) Formation of formate is a rapid reaction, but is limited by thermodynamic equilibrium, while hydrogenation of formate on the other hand is only limited by kinetics. Although the equilibrium limitation of alcoholate carbonylation was found to be lifted by consecutive removal of formate, the steady state formate concentration has appeared to be relatively low since the conditions are governed to a large extent by the requirements of the second step.

2) When a stoichiometric hydrogen/carbon monoxide synthesis gas mixture is converted at high methanol yield, the methanol concentration in the liquid catalyst phase has found to be relatively high under steady state conditions, even when originally a catalyst with a higher alcohol as alcoholate component has been applied.

It has been found, that methanol is a less suitable alcohol component for the formate ester formation than an optimally selected one e.g. selected from the group of alkanols having 4 to 10 carbon atoms as far as the reaction is concerned with reference to reaction rates, mass transfer, gas solubilisation etc. Moreover, at high methanol concentrations methylformate will inevitably be formed in competition with other formates.

Since methylformate is relatively volatile, it will be a substantial byproduct of the methanol produced unless the steady state formate concentration can be kept deliberately low, which is not inductive to a high hydrogenation rate. It will be appreciated that maintaining the earlier conception of a single stage process, significant potential activity is sacrificed. Even if methyl formate is accepted as byproduct, additional costs will be incurred for its separation from methanol and to revert it subsequently to methanol.

As component (c) of the catalyst system a hydride of lithium, sodium, potassium, rubidium, cesium, calcium, strontium, basium or magnesium may be used. Preference is given to sodium hydride.

The alcohol of component (b) may be cycloaliphatic or aliphatic, but is preferably aliphatic. Preference is given to alkanols, in particular to those having from 1 to 20 carbon atoms per molecule. Among the latter alkanols those having from 4 to 20 carbon atoms per molecule are preferred. Tertiary alcohols are more preferred. Examples of more preferred alkanols are tert-butyl alcohol and tert-pentyl alcohol. However also hexanol, heptanol and alkanols having from 8 to 20 carbon atoms per molecule may be used.

Dihydric alcohols may also be used, for example ethylene glycol, propylene glycol, 1,3-dihydroxypropane, 1,2-butanediol, 1,3-butanediol, 1,4-butanediol, 2,3-butanediol or 1,2-pentanediol. Component (b) may also be glycerol.

Component (b) may be a mixture of alcohols, for example of tert-butyl alcohol and ethylene glycol or of tert-phenyl alcohol and 1,4-butanediol.

The alcoholate to be used is preferably a sodium alcoholate or a potassium alcoholate. Among the alcoholates preference is given to alkoxides, particularly to those having from 1 to 20 carbon atoms per molecule, such as sodium methoxide, sodium ethoxide, sodium propoxide, sodium butoxide, sodium isobutoxide, sodium tert-pentoxide and potassium 2-methyldode-2-oxide.

The anion of the salt in component (a) may be derived from a great variety of acids. It is preferred that the salt in component (a) is a salt of a carboxylic acid or sulphonic acids. Among these acids preference is given to alkanoic acids having 1-10 carbon atoms in the chain or to paratoluene sulphonic acid. More preference is given to formic acid, acetic acid and oxalic acid. Component (a) is most preferably nickel formate, nickel acetate, nickel oxalate or nickel tosylate.

The salts in component (a) may contain crystal water, but are preferably free therefrom.

Activation of the catalyst system to be used can be reached by a great variety of methods as will be understood by the person skilled in the art.

The activation of the catalyst system, which has appeared to provide attractive results, may be reached by keeping the mixed components under an atmosphere of nitrogen or any other suitable inert gas during 0.3 to 1 hour at a temperature in the range of from 20-60 $^\circ$C and more preferably 35-50 $^\circ$C.

The process step predominantly comprising the carbonylation reaction, may be carried out at a temperature in the range of from 20-70 $^\circ$C and more preferably in the range of from 30-50 $^\circ$C, while the process step predominantly comprising the hydrogenation reaction, may be carried out at a temperature in the range of from 70-150 $^\circ$C and more preferably in the range of from 80-100 $^\circ$C.

If the total reaction heat may be removed from the circulating catalyst as depicted in the figure, the temperature difference will almost automatically be obtained.

The present process has a much greater flexibility than a single stage conversion of syngas and offers extensive opportunities for optimization enabling the separated reaction steps at the desired different reaction temperatures, governed by the fact that carbonylation is a fast but equilibrium-limited reaction and needs a relatively low temperature, where the equilibria are more favourable, while the formate hydrogenation is not limited by equilibria but by kinetics, giving advantages by carrying out this step at higher temperatures.

In the carbonylation reaction zone a pressure is applied from 10-80 bar and preferably from 20-70 bar. In the hydrogenation reaction zone a pressure is applied from 10-80 bar and preferably from 20-70 bar.

The process according to the present invention may be in principle carried out with an organic diluent in which the catalytic system is dissolved or suspended, but preferably the reaction medium is formed in the alcohol in excess and/or alcoholate. Example of suitable diluents are ethers, especially 2,5,8-trioxanone, aromatic hydrocarbons, halogenated aromatic compounds, sulphones and mixtures thereof.

The carbon monoxide/hydrogen mixtures may be used as pure gases, but may also be used being diluted, for example with nitrogen. Most preferably gas mixtures are used obtained from conversion of natural gas or from coal gasification.

According to further optimized embodiments of the process of the present invention, provisions are made to achieve liquid stageing and countercurrent gas/liquid contacting by means generally known in the art, e.g. by plate/bubble caps construction or compartments packed with distributing and mass transfer facilitating bodies e.g. rings, flakes etc.

If such provisions are applied in one or both reaction zones, more an effective stripping of methanol product from the liquid phase in the second reaction zone will be possible, avoiding the built up of methanol in the catalyst in the second reactor zone and also the formate byproduct formation will be minimized.

It will be appreciated that each reaction step of the process of the present invention can be carried out in one or more in series coupled reactors.

The great advantage of the process of the present invention is formed by the greater degree of flexibility as compared to a single stage process, which leads to an overall much better performance, e.g. a lower total reactor volume, while also the problem of methanol build up and formate byproduct formation will be minimized.

It will be appreciated that according to a further embodiment of the present invention, the process may be carried out in case of smaller capacities in at least two reaction zones accommodated in one and same vessel with suitable internals to separate the two reaction zones.

## Claims

1. A process for the manufacture of methanol comprising reaction of a mixture comprising carbon monoxide and hydrogen in the presence of a catalyst system obtainable by combination of at least:
(a) a nickel salt,
(b) an alcohol and/or an alcoholate from an alkaline metal or alkaline earth metal, and
(c) a hydride of an alkali metal or an alkaline earth metal,
and allowing the combined components (a), (b) and (c) to react, in at least two separate interconnected reaction zones, between which the catalyst system is circulated, into which mixtures comprising carbon monoxide and hydrogen of different composition are introduced and which are operated under different process conditions.

2. A process according to claim 1, characterized in that in one reaction zone a lower alkyl formate is formed by reaction of the mixture comprising carbon monoxide and hydrogen which has a hydrogen-carbon monoxide ratio from 0.5 to 1.9, preferably from 1.0 to 1.8, while in the other reaction zone the formate is hydrogenated to methanol with a hydrogen-carbon monoxide mixture, having a molar ratio in the range of from 2.5 to 4.5, preferably from 2.9 to 3.2.

3. A process according to any one of the claims 1 and 2, characterized in that the gas feeds to the two separate reaction zones have been derived from natural gas by its conversion into a synthesis gas, relatively rich in carbon monoxide by partial oxidation and by its conversion into a synthesis gas, relatively rich in hydrogen, by steam reforming.

4. A process according to any one of the claims 1-3,characterized in that the formate formation reaction is carried out at a temperature in the range of from 20 to 70 °C, preferably from 30 to 50 °C.

5. A process according to any one of the claims 1-4,characterized in that the formate hydrogenation reaction is carried out at a temperature in the range of from 70 to 150 °C, preferably from 80 to 100 °C.

6. A process according to any one of the claims 1-5,characterized in that the formate ester formation reaction step and the formate ester hydrogenation step are carried out at a pressure in the range of from 20 to 70 bar.

7. A process according to any one of the claims 1-6,characterized in that as catalyst component (a) nickel formate, nickel acetate, nickel oxalate or nickel tosylate is used.

8. A process according to any one of the claims 1-7,characterized in that as catalyst component (b) tert butyl alcohol, tert pentyl alcohol and/or the corresponding sodium or potassium alcoholates are used.

9. A process according to any one of the claims 1-8, characterized in that as catalyst component (c) sodium hydride is used.

10. A process according to any one of the claims 1-9, characterized in that methyl formate is formed in one of the reaction zones, which is converted into methanol in the other reaction zone.

FIG.1

PRODUCT

FEED
GAS

$T_1$

$T_2$

$T_2 > T_1$

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-4 613 623 (D.MAHAJAN)<br>* Column 2, lines 16-59; column 4, claims * | 1 | C 07 C 31/04<br>C 07 C 29/15<br>C 07 C 29/136<br>C 07 C 69/06<br>C 07 C 67/36 |
| D,A | US-A-4 614 749 (R.S.SAPIENZA)<br>* Column 1, line 59 - column 2, line 62; column 6, claims * | 1 | |
| D,A | US-A-4 619 946 (R.S.SAPIENZA)<br>* Column 6, claims * | 1 | |

TECHNICAL FIELDS SEARCHED (Int. Cl.4)

C 07 C 29/00
C 07 C 31/00

The present search report has been drawn up for all claims

| Place of search | Date of completion of the search | Examiner |
|---|---|---|
| THE HAGUE | 07-11-1988 | KINZINGER J.M. |